# EUROPEAN PATENT APPLICATION

(11) **EP 4 586 268 A1**
(43) Date of publication of application: **16.07.2025**
(21) Application number: 24151374.6
(22) Date of filing: 11.01.2024
(51) Int. Cl.: G16H 40/60, G16H 40/63, A61N 5/10

(54) **METHOD FOR OPERATING A MEDICAL DEVICE, MEDICAL DEVICE AND COMPUTER PROGRAM**

(71) Applicant: Koninklijke Philips N.V., 5656 AG Eindhoven (NL)
(72) Inventor: WEISS, Steffen, Eindhoven (NL); VOGTMEIER, Gereon, Eindhoven (NL)
(74) Representative: Philips Intellectual Property & Standards

(57) **Abstract**

The invention relates to a method (6) for operating a medical device (2). According to the method (6), the medical device (2) communicates (S 1) with at least one electronic device (3; 4) about scheduling of emission of electric and/or magnetic fields. Further, the medical device (2) determines (S2) a timing for an action of the medical device (2) that leads to the emission of electric and/or magnetic fields and/or is sensitive to electric and/or magnetic fields. The steps of determining (S2) the timing and communicating (S1) with the at least one electronic device (3; 4) are performed in agreement (7) with one another. Finally, the medical device (2) performs (S3) the action of the medical device (2) based on the determined timing. The invention further relates to a corresponding medical device (2) and computer program.

## Description

### FIELD OF THE INVENTION

The invention relates to a medical environment, e.g., to a hospital or to a doctor's practice. In particular, the invention relates to a method for operating a medical device, to a corresponding medical device and to a corresponding computer program.

### BACKGROUND OF THE INVENTION

Medical institutions are equipped with various electric medical and non-medical devices which emit electromagnetic radiation. Said devices include typical medical equipment such as imaging and monitoring devices, private equipment such as mobile phones and computers, hospital installations such as elevators, and temporary construction devices such as welding machines. These devices may emit radiofrequency (RF) radiation, strong slowly varying magnetic fields (e.g., by power supplies) and/or ionization radiation (e.g., by X-ray-based imaging and nuclear medicine devices).

While electromagnetic emission of devices is limited by regulations, these regulations may be insufficient for the combination of two devices, especially, if they are closely neighbored. In particular, in many situations in a hospital, e.g., in densely equipped intensive care rooms, there is a need for simultaneous operation of devices in close proximity rather than solving the problem by switching off a device or going at distance. Additionally, many devices in a hospital environment are mobile, and medical staff is typically not aware of electromagnetic emission and interference between devices and its strong dependence on distance. As a result, electromagnetic emission in many cases still impairs function and safety of devices.

As an example, mobile medical devices are often brought into magnetic resonance (MR) suites despite permission to do so and cause MR image artefacts. Likewise, the RF emission of the MR system may impair function and safety of these mobile devices. As another example, X-ray imaging systems may emit direct and scatter radiation that can interfere with sensors by creating noise, e.g., in form of "colored snow" in camera images.

Furthermore, limiting emission and impact of electromagnetic radiation requires considerable design and construction effort, e.g., for shielding or filtering, both in hardware and software. In some cases, even functionality of devices must be limited in order to comply with regulations or to cope with incoming electromagnetic radiation. Currently, being compliant with regulations for electromagnetic emission and applying the still required effort to shield and filter devices is the most practical approach to achieve compatibility of any two devices at any distance.

### SUMMARY OF THE INVENTION

It is therefore an object of the present invention to improve on dealing with electromagnetic radiation. In particular, it is an object of the present invention to provide an improved method for operating a medical device. It is a further object of the present invention to provide a related medical device and a related computer program.

The object of the present invention is solved by the subject matter of the independent claims, wherein further embodiments are incorporated in the dependent claims.

In an aspect of the present invention, a method for operating a medical device is provided. Said medical device may be a medical device that emits electric and/or magnetic fields and/or is sensitive to electric and/or magnetic fields.

The method comprises communicating, by the medical device, with at least one electronic device about scheduling of emission of electric and/or magnetic fields. Said communication may also be referred to as exchange of information. The communication may be performed via wireless channels, such as Bluetooth, Wi-Fi, or Wi-Fi direct, or via wired channels, such as a hospital Ethernet. The at least one electronic device is, in particular, different from the medical device.

Further, the method comprises determining, by the medical device, a timing for an action of the medical device that leads to the emission of electric and/or magnetic fields and/or is sensitive to electric and/or magnetic fields. If the action of the medical device leads to the emission of electric and/or magnetic fields, the purpose of the method is to ease the effects of the emission of electric and/or magnetic fields by the medical device. If, on the other hand, the action of the medical device is sensitive to electric and/or magnetic fields, the purpose of the method is to ease the effects of an emission of electric and/or magnetic fields on the medical device. Of course, the purpose of the method may be a combination of the two above-mentioned purposes.

It is to be noted that the steps of communicating, by the medical device, with the at least one electronic device, and determining, by the medical device, a timing for an action of the medical device may be in either order, i.e., the step of communicating may be performed first and the step of determining afterwards, or the step of determining may be performed first, and the step of communicating afterwards, or the two steps may be performed concurrently.

In either case, the steps of determining the timing and communicating with the at least one electronic device are performed in agreement with one another. That is, the determination of the timing may agree with the results of the communication, i.e., depend on the results of the communication, the communication may agree with the results of the determination, i.e., depend on the determination, and/or the determination of the timing and the communication may be interdependent. In this context, "in agreement with one another" means that the communication and/or determination of the timing will help easing the effects of the emission of electric and/or magnetic fields, by the medical device and/or on the medical device.

Finally, the action of the medical device is performed based on the determined timing. That is, the action of the medical device is performed at the determined timing. By performing the action of the medical device at the determined timing, the effects of the emission of electric and/or magnetic fields by the medical device and/or on the medical device are reduced and the method for operating the medical device is improved.

According to an embodiment, the medical device is a medical imaging device such as an X-ray imaging device, a computed tomography (CT) device, or a magnetic resonance imaging (MRI) device. More particularly, the medical device may be a mobile imaging system. Alternatively, or additionally, the medical device is a medical treatment device, such as an image guided therapy (IGT) device. All of these medical devices both emit electric and/or magnetic fields and are sensitive to electric and/or magnetic fields, hence the operation of these medical devices is greatly improved by the described method.

According to an embodiment, the at least one electronic device comprises another medical device, a computing device, and/or an infrastructure device. The other medical device may be another medical imaging device, a patient monitoring system, and/or a life-support system. The computing device may be a stationary computing device, a mobile computing device, and/or a handheld computing device, such as a smartphone. The computing devices may have an app installed thereon that enables the communication with the medical device. The infrastructure device may be an air conditioning system, an elevator, and/or a cleaning system.

Alternatively, or additionally, the at least one electronic device comprises a management device that communicates with at least one other electronic device. Said management device may be a device management device of the medical environment, e.g., of the doctor's practice or of the hospital. The other electronic device may be yet another medical device, another computing device, and/or another infrastructure device. In this case, the communication of the medical device with the other electronic device may be via the management device.

According to an embodiment, the electric and/or magnetic fields comprise radiofrequency electromagnetic fields, magnetic fields, and/or X-rays. Fields like the magnetic fields may be static, slowly varying, or rapidly varying. Any of these electric and/or magnetic fields may disturb the at least one electronic device and/or the at least one other electronic device and/or the medical device, hence the effects of all of the above-mentioned electric and/or magnetic fields may be considered.

According to an embodiment, the step of communicating with the at least one electronic device comprises sending information to the at least one electronic device and/or receiving information from the at least one electronic device. Hence, the communication may be uni-directional (either from the medical device or to the medical device) and broadcast-like or bi-directional (from and to the medical device) and hand-shake-like. Said communication may further involve setting of device clocks, or aligning on time differences between device clocks (in case the device clocks shall not be changed). Hence, several communication options are possible and the timing of the action is not effected by inaccurate device clocks.

According to an embodiment, the steps of communicating with the at least one electronic device and determining the timing comprise setting, by the medical device, a timing for the action of the medical device, and sending, by the medical device, information on the set timing to the at least one electronic device. Hence, the medical device sets the timing of the action itself and informs the at least one electronic device about it, such that the at least one electronic device may take measures to reduce the effect of the emission of electric and/or magnetic fields. As an example, the sent information may be a warning broadcast with the planned timing and type of its emission (e.g., emission in the radiofrequency (RF) band from 63.8 to 64.2 MHz at 0dBm at a specific location for 2 min in a regular pattern of: RF on for 3ms and off for 7ms).

According to an embodiment, the method further comprises receiving, by the at least one electronic device, the information on the set timing. Based on said information, the at least one electronic device and/or the at least one other electronic device reduces an emission of electric and/or magnetic fields during the time period corresponding to the set timing. In this case, the information may include a request for pausing emissions of electric and/or magnetic fields, or certain emissions of electric and/or magnetic fields (e.g., emission in the RF band within a certain frequency range) by the at least one electronic device and/or the at least one other electronic device. Hence, by reducing and/or pausing the emission of electric and/or magnetic fields during the time periods when the action is performed by the medical device, disturbances of said electric and/or magnetic fields on the medical device are reduced or even eliminated, hence the medical device may operate more efficiently and/or accurately.

Alternatively, or additionally, the at least one electronic device and/or the at least one other electronic device adapts its actions based on the received information on the set timing. For example, the at least one electronic device and/or the at least one other electronic device my schedule their sensitive actions for times when the medical device does not emit electric and/or magnetic fields. That is, the at least one electronic device and/or the at least one other electronic device may use the information on the timing of the actions of the medical device to interleave their own critical (sub-) functions in time. An example may be to interleave own data acquisition with the RF emission of a mobile imaging system. Alternatively, or additionally, the at least one electronic device and/or the at least one other electronic device may use the knowledge of emission-free times (of the medical device) to apply filtering of data only if it is needed. That is, filtering is applied when the medical device performs the action, and no filtering is applied at other times. Also, especially software-based filters can be adapted by including the knowledge of type and timing of a foreign emission. Hence, data quality is improved and filtering effort is reduced.

According to an embodiment, the steps of communicating with the at least one electronic device and determining the timing comprise receiving, by the medical device, information from the at least one electronic device related to a timing of emission of electric and/or magnetic fields by the at least one electronic device and/or by the at least one other electronic device. Hence, the at least one electronic device and/or the at least one other electronic device sets the timing of the emission of electric and/or magnetic fields and informs the medical device about it. Based on said received information, the medical device sets the timing of the action of the medical device. In particular, the medical device sets the timing of the action to a time period where there is little or no emission of electric and/or magnetic fields by other devices, i.e., to interleave its action with the emission of electric and/or magnetic fields by the other devices, such that the action of the medical device may be performed efficiently and accurately.

According to an embodiment, the steps of communicating with the at least one electronic device and determining the timing comprise negotiating, by the medical device, with the at least one electronic device timings of emission of electric and/or magnetic fields. As an example, said negotiation may be performed with a management device, wherein the management device distributes the timings for emission of electric and/or magnetic fields. As another example, the negotiation may be performed with at least one electronic device and/or at least one other electronic device, wherein the at least one electronic device and/or the at least one other electronic device account for most of the emission of electric and/or magnetic fields in the vicinity of the medical device. Hence, in this case, two-way communication is performed to align the emission of electric and/or magnetic fields. For said negotiation, priorities of the devices may be used. Said priorities may be predetermined for a type of a device or for a specific device. The priorities may also be adjusted by the device, e.g., based on parameters of the device or of another device (e.g., a life-support system may have varying priorities based on the state of the patient, as given, e.g., by pulse, blood pressure, or oxygen saturation). Alternatively, or additionally, the priorities may be input by a user. Hence, an optimal distribution of emission timings between the medical device, the at least one electronic device and/or the at least one other electronic device may be achieved. Once the negotiating has been completed, the timing of the action of the medical device is set based on the results of the negotiation.

According to an embodiment, the method further comprises issuing, by the medical device, trigger signals relating to the timing of the action of the medical device. Information about these trigger signals may be already included in the information sent by the medical device. As an example, a mobile imaging system may announce in a warning broadcast that trigger signals will be issued that indicate the exact start time of the scheduled emission. The other devices receive both, the upfront scheduling information and the subsequent exactly timed triggers, to obtain an improved timing.

Likewise, the other devices, i.e., the at least one electronic device and/or the at least one other electronic device may emit trigger signals. As an example, the mobile imaging system may request pausing RF emission by other devices in its receive band. It may then wait for a trigger signal that marks the start of this "RF silence".

According to an embodiment, the method further comprises adapting the action of the medical device based on received information from the at least one electronic device. By this adaptation, the interfering emission of electric and/or magnetic fields by the at least one electronic device and/or at least one other electronic device is dealt with. In particular, the medical device may use the knowledge of emission-free times of the other devices to apply filtering of data only if it is needed. Also, especially software-based filters can be adapted by including the knowledge of type and timing of foreign emission. Hence, data quality is improved and/or filtering effort is reduced.

According to an embodiment, the method further comprises determining at least one distance between the medical device and the at least one electronic device and/or the at least one other electronic device, respectively. This distance may be determined by using known positions of the device, e.g., within the medical environment. Alternatively, the distance may be determined from the strength of a wireless signal that is used for the communication. When the distance has been determined, at least one of the steps of determining the timing for the action of the medical device; reducing the emission of electric and/or magnetic fields; adapting the actions of the at least one electronic device and/or of the at least one other electronic device; setting the timing of the action of the medical device; and/or adapting the action of the medical device is performed based on the determined at least one distance. In particular, emissions of electric and/or magnetic fields by other devices may be neglected is said other devices are too far away from the medical device. In other words, the above steps may be only applied if two devices are in a mutual interference range. Hence, unnecessary emission limitations are avoided.

According to an embodiment, the method further comprises logging the communication of the medical device with the at least one electronic device and analyzing the logged communication to identify critical regions and/or critical times. As an example, the more critical signals are sent in a hospital the more likely is the chance that non-compliant devices will suffer from interferences. As another example, the logging information may be used to identify the risks for older devices that are not (yet) compatible with the above-described method. As yet another examples, the logging of the messages and a statistical processing of the logged messages may help identifying critical regions and critical times as an early warning for risky situations. As yet another example, the logging and processing may also detect abnormal behavior of devices, e.g., in case of too often transmitted activity information, the device might have a problem or does not work properly in the hospital network.

In another aspect of the present invention, a medical device is provided. The medical device comprises a computing unit and is configured to carry out the method according to the above description. Hence, details, further embodiments and advantages of the medical device correspond to those of the above-described method for operating a medical device.

In yet another aspect of the present invention, a computer program is provided. When the computer program is executed by a computer, it causes the computer to carry out the method according to the above description. Hence, details, further embodiments and advantages of the computer program correspond to those of the above-described method for operating a medical device.

These and other aspects of the invention will be apparent from and elucidated with reference to the embodiments described hereinafter.

### BRIEF DESCRIPTION OF THE DRAWINGS

In the following, preferred embodiments of the invention will be described, by way of example only, and with reference to the drawings in which:
Fig. 1 shows a schematic view of an embodiment of a medical environment;
Fig. 2 shows a flowchart of an embodiment of a method for operating a medical device;
Fig. 3 shows a flowchart of an embodiment of another method for operating a medical device;
Fig. 4 shows a flowchart of an embodiment of yet another method for operating a medical device; and
Fig. 5 shows a flowchart of an embodiment of yet another method for operating a medical device.

In the Figures, elements which correspond to elements already described may have the same reference numerals. Examples, embodiments or optional features, whether indicated as non-limiting or not, are not to be understood as limiting the invention as claimed.

### DETAILED DESCRIPTION OF EMBODIMENTS

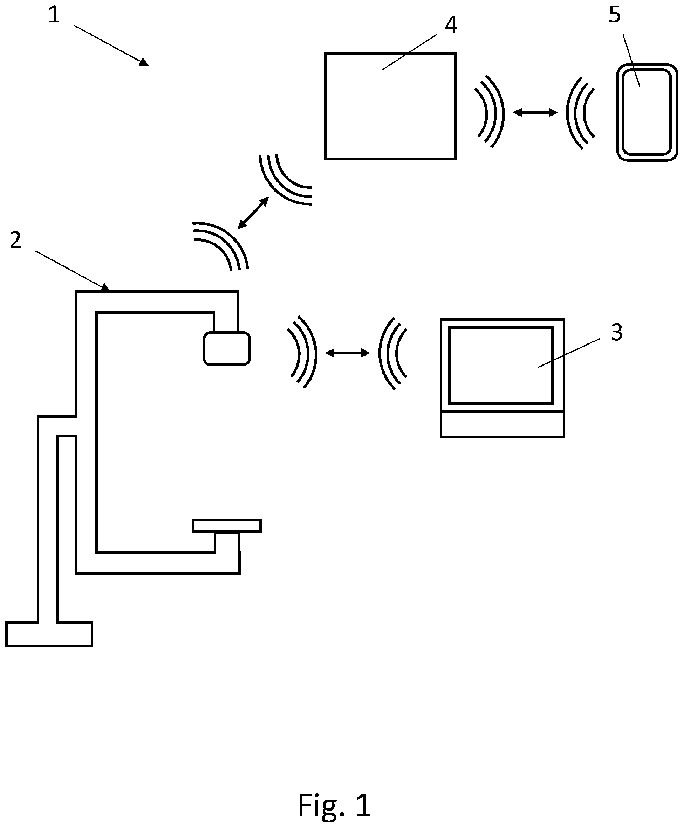

Fig. 1 shows a schematic view of an embodiment of a medical environment 1. The medical environment 1 may, e.g., be a doctor's practice or a hospital.

The medical environment 1 comprises a medical device 2. In the Figure, the medical device 2 is shown as a mobile C-arm X-ray imaging system, however, the medical device 2 may be another medical imaging device such as an X-ray imaging device, a computed tomography (CT) device, or a magnetic resonance imaging (MRI) device or a medical treatment device, such as an image guided therapy (IGT) device. All of these medical devices 2 both emit electric and/or magnetic fields and are sensitive to electric and/or magnetic fields.

The medical environment 1 further comprises a patient monitoring system 3 and a management device 4 as examples for two electronic devices. Additionally, or alternatively, the medical environment 1 may comprise other electronic devices such as other medical devices, computing devices, and/or infrastructure devices.

The medical device 2 and the electronic devices 3 and 4 are configured to communicate with one another, via a wireless (as indicated in the Figure) and/or a wired connection.

The management device 4 is further configured to communicate with other electronic devices such as a computing device 5, shown as a smartphone in the Figure.

Moreover, the medical device 2 is configured to perform the method described below.

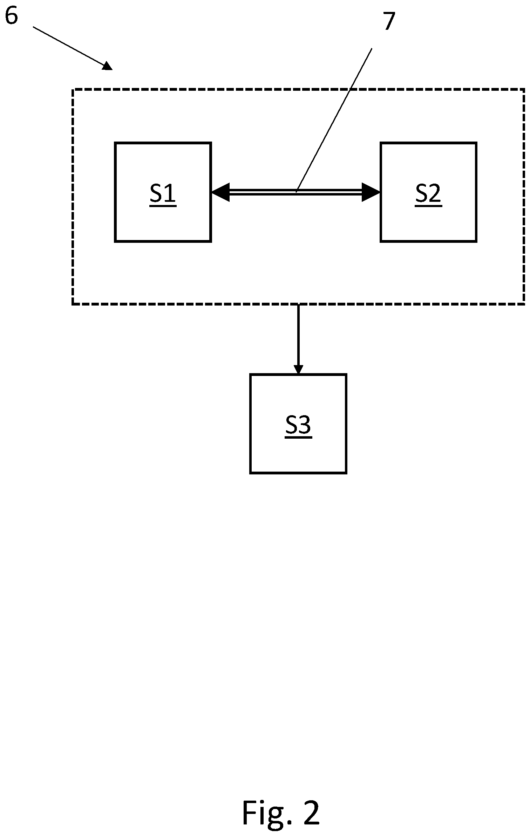

Fig. 2 shows a flowchart of an embodiment of a method 6 for operating a medical device 2. The method 6 comprises communicating S1, by the medical device 2, with at least one electronic device 3; 4 about scheduling of emission of electric and/or magnetic fields. Said communication may also be referred to as exchange of information. The communication may be performed via wireless channels, such as Bluetooth, Wi-Fi, or Wi-Fi direct, or via wired channels, such as a hospital Ethernet. The at least one electronic device 3; 4 is, in particular, different from the medical device 2.

Further, the method 6 comprises determining S2, by the medical device 2, a timing for an action of the medical device 2 that leads to the emission of electric and/or magnetic fields and/or is sensitive to electric and/or magnetic fields. If the action of the medical device 2 leads to the emission of electric and/or magnetic fields, the purpose of the method 6 is to ease the effects of the emission of electric and/or magnetic fields by the medical device 2. If, on the other hand, the action of the medical device 2 is sensitive to electric and/or magnetic fields, the purpose of the method is to ease the effects of an emission of electric and/or magnetic fields on the medical device 2. Of course, the purpose of the method 6 may be a combination of the two above-mentioned purposes.

It is to be noted that the steps of communicating S 1, by the medical device 2, with the at least one electronic device 3; 4, and determining S2, by the medical device 2, a timing for an action of the medical device 2 may be in either order, i.e., the step of communicating S1 may be performed first and the step of determining S2 afterwards, or the step of determining S2 may be performed first, and the step of communicating S1 afterwards, or the two steps S 1; S2 may be performed concurrently.

In either case, the steps of determining S2 the timing and communicating S1 with the at least one electronic device 3; 4 are performed in agreement 7 with one another, as indicated by the arrow. That is, the determination S2 of the timing may agree with the results of the communication S 1, i.e., depend on the results of the communication S 1, the communication S1 may agree with the results of the determination S2, i.e., depend on the determination S2, and/or the determination S2 of the timing and the communication S 1 may be interdependent. In this context, "in agreement 7 with one another" means that the communication S1 and/or determination S2 of the timing will help easing the effects of the emission of electric and/or magnetic fields, by the medical device 2 and/or on the medical device 2.

Finally, the action of the medical device 2 is performed S3 based on the determined S2 timing. That is, the action of the medical device 2 is performed at the determined S2 timing. By performing the action of the medical device 2 at the determined S2 timing, the effects of the emission of electric and/or magnetic fields by the medical device 2 and/or on the medical device 2 are reduced and the method 6 for operating the medical device 2 is improved.

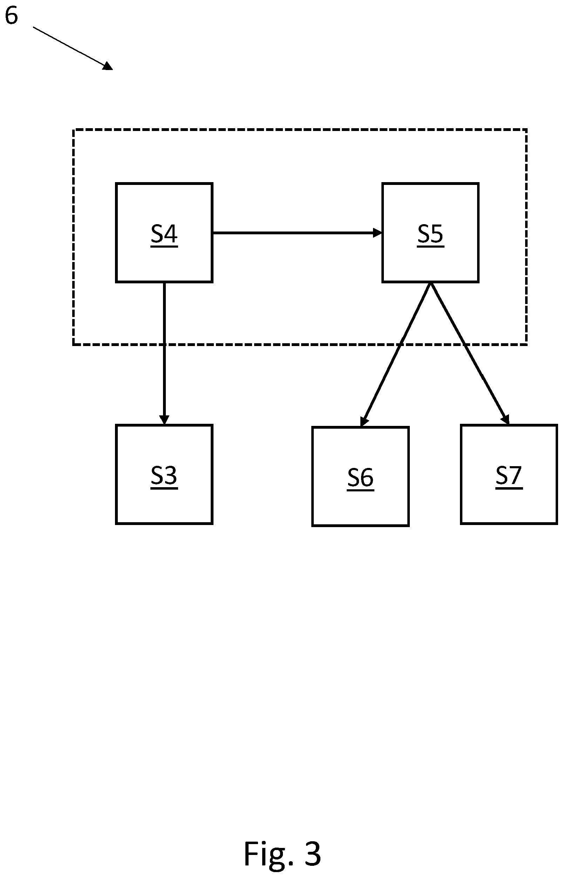

Fig. 3 shows a flowchart of an embodiment of another method 6 for operating a medical device 2. The steps of communicating S1 with the at least one electronic device 3; 4 and determining S2 the timing (as indicated by the dashed box) comprise setting S4, by the medical device 2, a timing for the action of the medical device 2, and sending S5, by the medical device 2, information on the set timing to the at least one electronic device 3; 4. Hence, the medical device 2 sets the timing of the action itself and informs the at least one electronic device 3; 4 about it, such that the at least one electronic device 3; 4 may take measures to reduce the effect of the emission of electric and/or magnetic fields. One of said measures is to reduce S6, based on said information, by the at least one electronic device 3; 4 and/or the at least one other electronic device 5 an emission of electric and/or magnetic fields during the time period corresponding to the set timing. In this case, the information may include a request for pausing emissions of electric and/or magnetic fields, or certain emissions of electric and/or magnetic fields (e.g., emission in the RF band within a certain frequency range) by the at least one electronic device 3; 4 and/or the at least one other electronic device 5. Hence, by reducing S6 and/or pausing the emission of electric and/or magnetic fields during the time periods when the action is performed by the medical device 2, disturbances of said electric and/or magnetic fields on the medical device 2 are reduced or even eliminated, hence the medical device 2 may operate more efficiently and/or accurately. Alternatively, or additionally, the at least one electronic device 3; 4 and/or the at least one other electronic device 5 adapts S7 its actions based on the received information on the set timing. For example, the at least one electronic device 3; 4 and/or the at least one other electronic device 5 my schedule their sensitive actions for times when the medical device 2 does not emit electric and/or magnetic fields. That is, the at least one electronic device 3; 4 and/or the at least one other electronic device 5 may use the information on the timing of the actions of the medical device 2 to interleave their own critical (sub-) functions in time. An example may be to interleave own data acquisition with the RF emission of a mobile imaging system. Alternatively, or additionally, the at least one electronic device 3; 4 and/or the at least one other electronic device 5 may use the knowledge of emission-free times (of the medical device 2) to apply filtering of data only if it is needed. That is, filtering is applied when the medical device 2 performs the action, and no filtering is applied at other times. Also, especially software-based filters can be adapted by including the knowledge of type and timing of a foreign emission. Hence, data quality is improved and filtering effort is reduced.

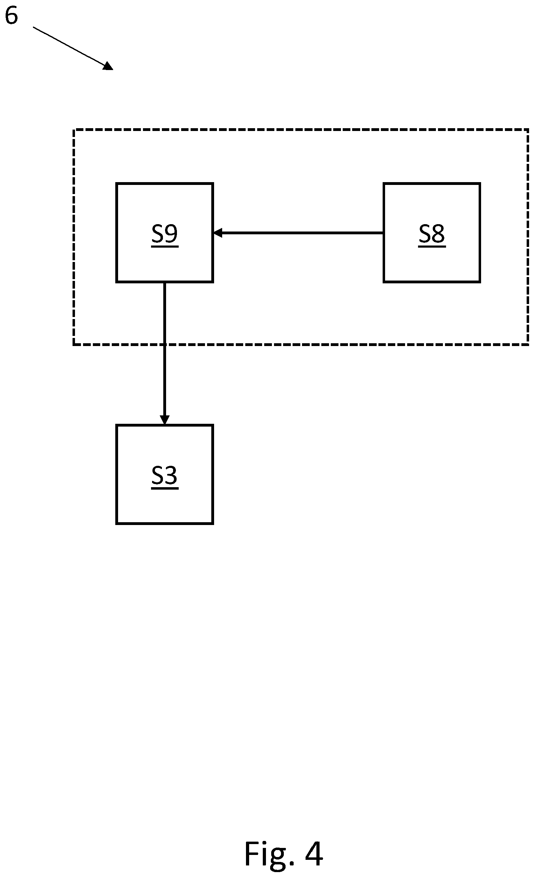

Fig. 4 shows a flowchart of an embodiment of yet another method 6 for operating a medical device 2. In this method 6, the steps of communicating S 1 with the at least one electronic device and determining S2 the timing (indicated by the dashed box) comprise receiving S8, by the medical device 2, information from the at least one electronic device 3; 4 related to a timing of emission of electric and/or magnetic fields by the at least one electronic device 3; 4 and/or by the at least one other electronic device 5. Hence, the at least one electronic device 3; 4 and/or the at least one other electronic device 5 sets the timing of the emission of electric and/or magnetic fields and informs the medical device 2 about it. Based on said received S8 information, the medical device 2 sets S9 the timing of the action of the medical device 2. In particular, the medical device 2 sets the timing of the action to a time period where there is little or no emission of electric and/or magnetic fields by other devices 3; 4; 5, i.e., to interleave its action with the emission of electric and/or magnetic fields by the other devices 3; 4; 5, such that the action of the medical device 2 may be performed efficiently and accurately.

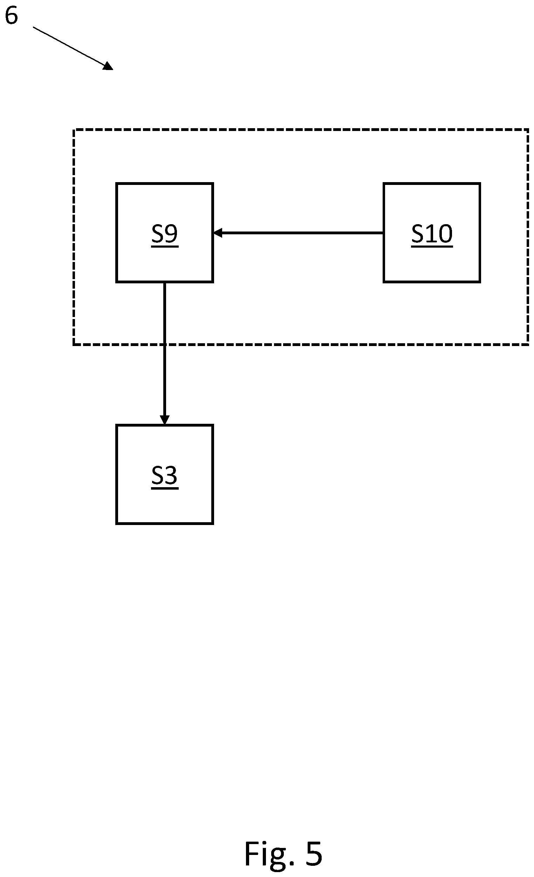

Fig. 5 shows a flowchart of an embodiment of yet another method 6 for operating a medical device 2. In this method 6, the steps of communicating S 1 with the at least one electronic device 3; 4 and determining S2 the timing (as indicated by the dashed box) comprise negotiating S10, by the medical device 2, with the at least one electronic device 3; 4 timings of emission of electric and/or magnetic fields. As an example, said negotiation S 10 may be performed with a management device 4, wherein the management device 4 distributes the timings for emission of electric and/or magnetic fields. As another example, the negotiation S10 may be performed with at least one electronic device 3; 4 and/or at least one other electronic device 5, wherein the at least one electronic device 3; 4 and/or the at least one other electronic device 5 account for most of the emission of electric and/or magnetic fields in the vicinity of the medical device 2. Hence, in this case, two-way communication is performed to align the emission of electric and/or magnetic fields. For said negotiation, priorities of the devices may be used. Said priorities may be predetermined for a type of a device or for a specific device. The priorities may also be adjusted by the device, e.g., based on parameters of the device or of another device (e.g., a life-support system may have varying priorities based on the state of the patient, as given, e.g., by pulse, blood pressure, or oxygen saturation). Alternatively, or additionally, the priorities may be input by a user. Hence, an optimal distribution of emission timings between the medical device 2, the at least one electronic device 3; 4 and/or the at least one other electronic device 5 may be achieved. Once the negotiating S 10 has been completed, the timing of the action of the medical device 2 is set S9 based on the results of the negotiation S 10.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

### LIST OF REFERENCE SIGNS:

- 1: medical environment
- 2: medical device
- 3: monitoring system
- 4: management device
- 5: computing device
- 6: method
- 7: agreement

- S1: communicating
- S2: determining
- S3: performing
- S4: setting
- S5: sending
- S6: reducing
- S7: adapting
- S8: receiving
- S9: setting
- S10: negotiating

## Claims

1. A method (6) for operating a medical device (2), comprising:
communicating (S 1), by the medical device (2), with at least one electronic device (3; 4) about scheduling of emission of electric and/or magnetic fields;
determining (S2), by the medical device (2), a timing for an action of the medical device (2) that leads to the emission of electric and/or magnetic fields and/or is sensitive to electric and/or magnetic fields,
wherein the steps of determining (S2) the timing and communicating (S 1) with the at least one electronic device (3; 4) are performed in agreement (7) with one another; and
performing (S3), by the medical device (2), the action of the medical device (2) based on the determined timing.

2. The method (6) according to claim 1, wherein the medical device (2) is a medical imaging device, more particularly a mobile imaging system, and/or a medical treatment device.

3. The method (6) according to claim 1 or 2, wherein the at least one electronic device (3; 4) comprises another medical device (3), a computing device, an infrastructure device, and/or a management device (4) communicating with at least one other electronic device (5).

4. The method (6) according to any one of claims 1 to 3, wherein the electric and/or magnetic fields comprise radiofrequency electromagnetic fields, magnetic fields, and/or X-rays.

5. The method (6) according to any one of claims 1 to 4, wherein communicating (S1) with the at least one electronic device (3; 4) comprises sending information to the at least one electronic device (3; 4) and/or receiving information from the at least one electronic device (3; 4).

6. The method (6) according to any one of claims 1 to 5, wherein communicating (S1) with the at least one electronic device (3; 4) and determining (S2) the timing comprises:
setting (S4), by the medical device (2), a timing for the action of the medical device (2); and
sending (S5), by the medical device (2), information on the set timing to the at least one electronic device (3; 4).

7. The method (6) according to claim 6, further comprising:
receiving, by the at least one electronic device (3; 4), the information on the set timing; and
reducing (S6), by the at least one electronic device (3; 4) and/or by the at least one other electronic device (5), an emission of electric and/or magnetic fields during the time period corresponding to the set timing; and/or
adapting (S7), by the at least one electronic device (3; 4) and/or by the at least one other electronic device (5), actions of the at least one electronic device (3; 4) and/or of the at least one other electronic device (5) based on the received information on the set timing.

8. The method (6) according to any one of claims 1 to 7, wherein communicating (S1) with the at least one electronic device (3; 4) and determining (S2) a timing comprises:
receiving (S8), by the medical device (2), information from the at least one electronic device (3; 4) relating to a timing of emission of electric and/or magnetic fields by the at least one electronic device (3; 4) and/or by the at least one other electronic device (5); and
setting (S9), by the medical device (2), the timing of the action of the medical device (2) based on the received information.

9. The method (6) according to any one of claims 1 to 8, wherein communicating (S1) with the at least one electronic device (3; 4) and determining (S2) a timing comprises:
negotiating (S10), by the medical device (2), with the at least one electronic device (3; 4) timings of emission of electric and/or magnetic fields; and
setting (S9) the timing of the action of the medical device (2) based on results of the negotiation.

10. The method (6) according to any one of claims 1 to 9, further comprising:
issuing, by the medical device (2), trigger signals relating to the timing of the action of the medical device (2).

11. The method (6) according to any one of claims 1 to 10, further comprising:
adapting the action of the medical device (2) based on received information from the at least one electronic device (3; 4).

12. The method (6) according to any one of claims 1 to 11, further comprising:
determining at least one distance between the medical device (2) and the at least one electronic device (3; 4) and/or the at least one other electronic device (5), respectively, wherein at least one of the steps of
determining (S2) the timing for the action of the medical device (2); reducing (S6) the emission of electric and/or magnetic fields;
adapting (S7) the actions of the at least one electronic device (3; 4) and/or of the at least one other electronic device (5);
setting (S9) the timing of the action of the medical device (2); and/or
adapting the action of the medical device (2)
is performed based on the determined at least one distance.

13. The method (6) according to any one of claims 1 to 12, further comprising:
logging the communication of the medical device (2) with the at least one electronic device (3; 4); and
analyzing the logged communication to identify critical regions and/or critical times.

14. A medical device (2), comprising a computing unit, and configured to carry out the method (6) according to any one of claims 1 to 13.

15. A computer program comprising instructions which, when the program is executed by a computer, cause the computer to carry out the method (6) according to any one of claims 1 to 13.
